# EUROPÄISCHE PATENTSCHRIFT

(11) **EP 1 678 165 B1**
(45) Veröffentlichungstag und Bekanntmachung des Hinweises auf die Patenterteilung: **25.09.2013**
(21) Anmeldenummer: 04765927.1
(22) Anmeldetag: 12.10.2004
(51) Int. Cl.: C07D 405/12, A61K 31/505, A61P 35/00

(54) **VERFAHREN ZUR HERSTELLUNG VON AMINOCROTONYLVERBINDUNGEN**
METHOD FOR THE PRODUCTION OF AMINO CROTONYL COMPOUNDS
PROCEDE DE PRODUCTION DE COMPOSES AMINOCROTONYLIQUES

(30) Priorität: 17.10.2003 DE 10349113
(43) Veröffentlichungstag der Anmeldung: 12.07.2006
(60) Teilanmeldung: 12155662.5
(73) Patentinhaber: BOEHRINGER INGELHEIM INTERNATIONAL GMBH, 55216 Ingelheim am Rhein (DE); Boehringer Ingelheim Pharma GmbH & Co. KG, 55216 Ingelheim am Rhein (DE)
(72) Erfinder: RALL, Werner, 88441 MITTELBIBERACH (DE); SOYKA, Rainer, 88400 BIBERACH (DE); KULINNA, Christian, 88448 ATTENWEILER (DE); SCHNAUBELT, Juergen, 88447 Oberhöfen (DE); SIEGER, Peter, 88441 MITTELBIBERACH (DE)
(74) Vertreter: Simon, Elke Anna Maria
(86) Internationale Anmeldenummer: PCT/EP2004/011378
(87) Internationale Veröffentlichungsnummer: WO 2005/037824

(56) Entgegenhaltungen:
- WO-A-00/51991
- WO-A-02/50043

## Beschreibung

Die Erfindung betrifft ein verbessertes Verfahren zur Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin. Dieses besitzt folgende Struktur: 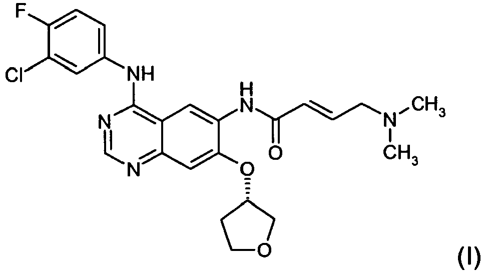 und ist bereits aus der WO 02/50043 bekannt, in der Verbindungen mit wertvollen pharmakologischen Eigenschaften beschrieben werden, zu denen insbesondere eine Hemmwirkung auf die durch Tyrosinkinasen vermittelte Signaltransduktion und eine Hemmwirkung auf die durch den Epidermal Growth Factor-Rezeptor (EGF-R) vermittelte Signaltransduktion gehören. Daher sind Verbindungen diese Typs zur Behandlung von Krankheiten, insbesondere zur Behandlung von Tumorerkrankungen, von Erkrankungen der Lunge und der Atemwege und von Erkrankungen des Magen-Darm-Traktes und der Gallengänge und -blase geeignet.

In der WO 02/50043 wird ein Herstellverfahren offenbart, bei dem Aminocrotonylverbindungen (IV) wie beispielsweise 4-[(3-Chlor-4-fluorphenyl)amino]-6-{[4-(N,Ndimethylamino)-1-oxo-2-buten-1-yl]amino}-7-((*S*)-tetrahydrofuran-3-yloxy)-chinazolin in einer Eintopfreaktion aus dem entsprechende Anilinbaustein (II), Bromcrotonsäure (III), Oxalylchlorid und einem sekundärem Amin hergestellt werden (s. Schema 1).

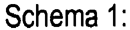

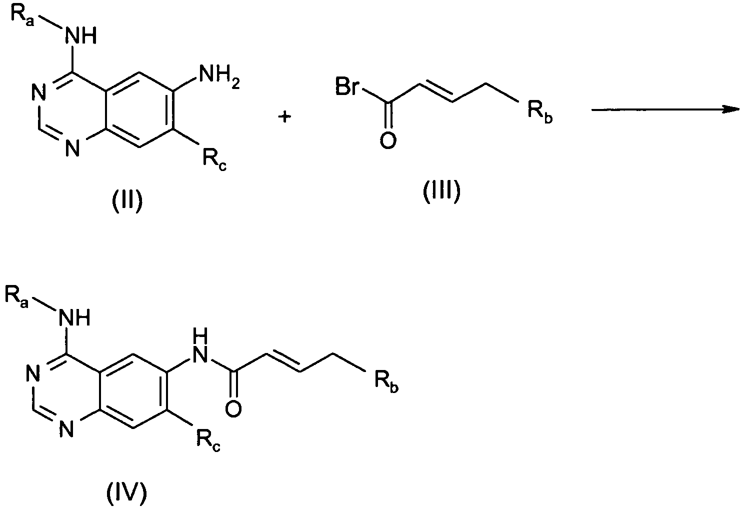

Bei diesem Verfahren lag die Ausbeute bei maximal 50 %. Zudem erfolgte die Reinigung in der Regel mittels Säulenchromatographie. Daher war das Verfahren für die Herstellung von 4-[(3-Chlor-4-fluorphenyl)amino]-6-{[4-(N,N-dimethylamino)-1-oxo-2buten-1-yl]amino}-7-((*S*)-tetrahydrofuran-3-yloxy)-chinazolin im technischen Maßstab nicht geeignet. Des weiteren hatte die Methode den Nachteil, dass Bromcrotonsäure in größeren Mengen nicht kommerziell erhältlich ist und auch der entsprechenden Bromcrotonsäuremethylester nur in ca. 80 %-iger Reinheit verfügbar ist. Diese Umstände widersprechen ebenfalls der Eignung dieses Verfahrens für die technische Herstellung von 4-[(3-Chlor-4-fluorphenyl)amino]-6-{[4-(N,N-dimethylamino)-1-oxo-2buten-1-yl]amino}-7-((*S*)-tetrahydrofuran-3-yloxy)-chinazolin.

Im Lichte der oben beschriebenen Nachteile des bekannten Herstellungsverfahrens ist es die Aufgabe der vorliegenden Erfindung, ein Verfahren bereitzustellen, das die Herstellung von 4-[(3-Chlor-4-fluorphenyl)amino]-6-{[4-(N,N-dimethylamino)-1-oxo-2buten-1-yl]amino}-7-((*S*)-tetrahydrofuran-3-yloxy)-chinazolin, unter Verwendung leicht zugänglicher Ausgangsstoffe in hoher Reinheit und ohne großen technischen Aufwand erlaubt. Dieses neue Verfahren soll also auch für die Synthese im technischen Maßstab und damit für die kommerzielle Anwendung geeignet sein.

Diese Aufgabe wird durch das erfindungsgemäße Verfahren zur Herstellung von 4-[(3-Chlor-4-fluorphenyl)amino]-6-{[4-(N,N-dimethylamino)-1-oxo-2-buten-1-yl]amino}7-((*S*)-tetrahydrofuran-3-yloxy)-chinazolin gelöst. Neben der technischen Durchführbarkeit in hohen Ausbeuten sind sehr gute chemische Reinheiten und ein niedriger cis-Gehalt von unter 0,1 % weitere Vorteile des erfindungsgemäßen Syntheseweges.

Gemäß dem erfindungsgemäßen Verfahren wird die entsprechende Aminoarylverbindung (V) mit einer Di-(C₁₋₄-alkyl)-phosphonoessigsäure, bevorzugt mit Diethylphosphonoessigsäure, in geeigneten Lösungsmitteln, nach entsprechender Aktivierung bevorzugt mit 1,1-Carbonyldiimidazol, 1,1-Carbonylditriazol oder Propanphosphonsäureanhydrid, besonders bevorzugt mit 1,1-Carbonyldiimidazol, gemäß Schema 2 umgesetzt. Als Lösungsmittel können beispielsweise Tetrahydrofuran (THF), Dimethylformamid (DMF) oder Ethylacetat verwendet werden.

Die Aktivierung kann nach allen gängigen Möglichkeiten zu Amidknüpfung erfolgen, also beispielsweise mit 1,1-Carbonyldiimidazol, 1,1-Carbonylditriazol, DCC (N,N-Dicyclohexylcarbodiimid), EDC (N'-(Dimethylaminopropyl)-N-ethylcarbodiimid), TBTU O-(Benzotriazol-1-yl)-N,N,N',N'-tetramethyluroniumtetrafluroborat, Thiazolidin-2-thione oder durch Überführung in das entsprechende Säurechlorid, etwa mit Hilfe von Thionylchlorid. Gegebenefalls wird die Aktivierung unter Verwendung von organischen Basen wie Triethylamin oder Pyridin durchgeführt, wobei zusätzlich DMAP ( Dimethylaminopyridin) zugesetzt werden kann. Als Lösungsmitteln kommen DMF, THF, Essigester, Toluol, chlorierte Kohlenwasserstoffe oder deren Gemisch in Frage. 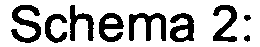 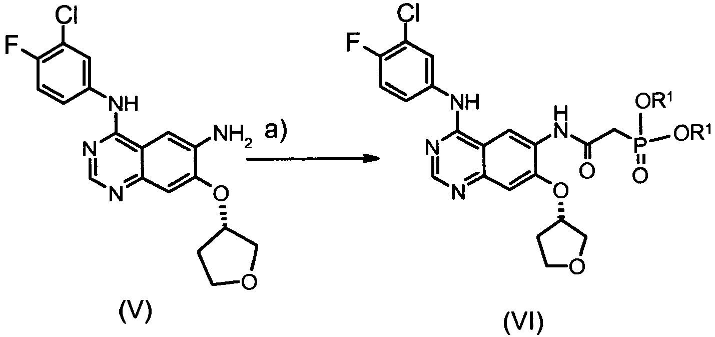
a) Di-(C₁₋₄-alkyl)-phosphonoessigsäure, Aktivierungsmittel
   Das so in hoher Ausbeute und hoher Reinheit erhaltene Arylamid (VI) wird mit dem entsprechenden 2-Aminoacetaldehyd unter Einsatz geeigneter organischer oder anorganischer Basen im Sinne einer Wittig-Horner-Emmons-Reaktion umgesetzt (Schema 3). Diese Umsetzung kann direkt oder nach Isolierung der Verbindung (VI), beispielsweise durch Ausfällen mittels Zugabe von beispielsweise *tert*-Butylmethylether, erfolgen. Zu den geeigneten Basen gehören beispielsweise DBU (1,5-Diaza-bicyclo[4.3.0]non-5-en), Natriumhydroxid und Kaliumhydroxid, bevorzugt sind Natriumhydroxid und Kaliumhydroxid, besonders bevorzugt ist Kaliumhydroxid. Anstelle des Aldehyds kann auch ein entsprechendes Äquivalent, z.B. ein Hydrat oder Acetal, aus dem der Aldehyd freigesetzt wird (vorab oder *in situ*), verwendet werden. 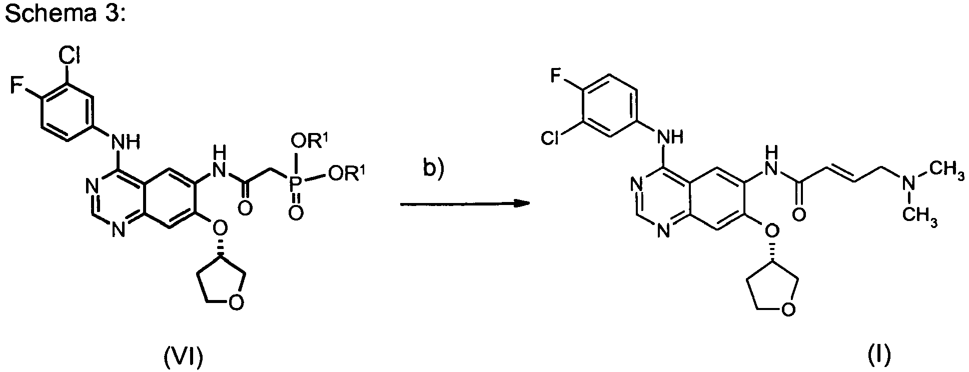
b) Aldehyd, Base, THF/Wasser
   Als Acetale können beispielsweise Verbindungen des folgenden allgemeinen Typs eingesetzt werden: 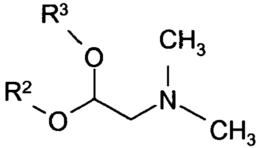 in denen R² und R³ jeweils eine geradkettige oder verzweigte C₁-C₄-Alkylgruppe bedeuten, wobei die Reste gleich oder verschieden sein können. Bevorzugt bedeuten R² und R³ jeweils eine Ethylgruppe.
   Das so erhaltene 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin der Formel (I) kann anschließend nach an sich bekannten Verfahren in seine Salze, insbesondere in seine physiologisch verträglichen Salze überführt werden.
   Die Ausgangsverbindung der Formel (V) kann beispielsweise wie folgt nach an sich literaturbekannten Verfahren hergestellt werden:
   Ausgehend von kommerziell erhältlicher 4-Chlor-anthranilsäure (VII; X = Cl) wird durch Umsetzung mit Formamidin-Acetat das Chinazolinon (VIII) erhalten, welches anschließend unter Verwendung von Schwefelsäure und konzentrierter Salpetersäure nitriert wird (Schema 4). Alternativ kann auch von 4-Fluor-anthranilsäure ausgegangen werden. 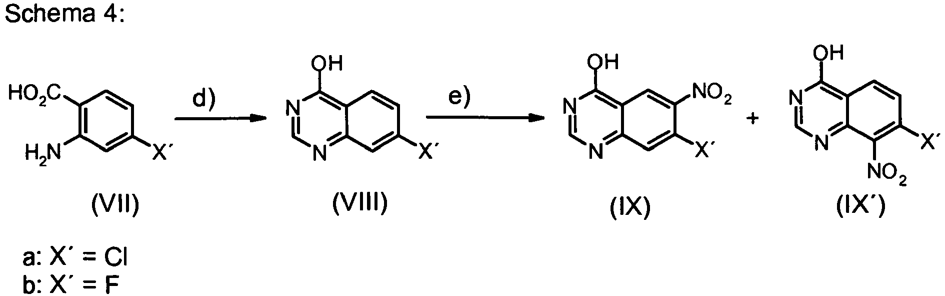
d) Formamidin-Acetat
e) H₂SO₄, HNO₃ konz.

Das gewünschte Regioisomer (IX) der so erhaltenen Nitrierungsprodukte wird dann chloriert, und das Chlorierungsprodukt (X) wird *in situ* mit dem entsprechendem Amin umgesetzt (Schema 6).

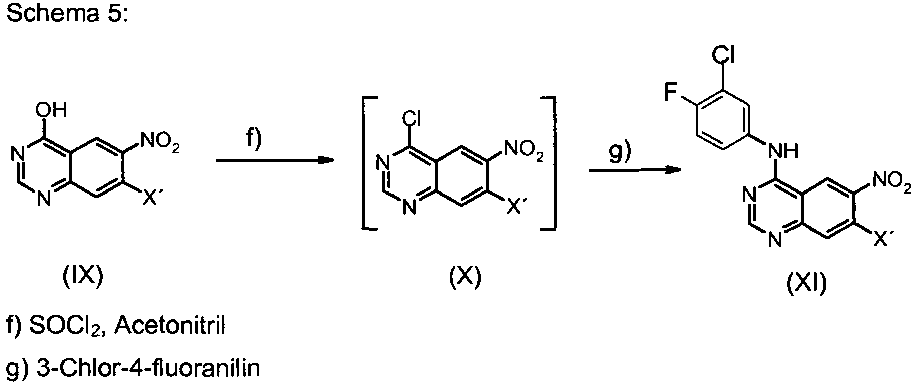

Die so erhaltene Verbindung der Formel (XI) wird mit (*S*)-(+)-3-Hydroxytetrahydro-furan zu Verbindung (XII) umgesetzt. Hydrierung der Verbindung (XII) ergibt dann die Ausgangsverbindung (V) (Schema 6).

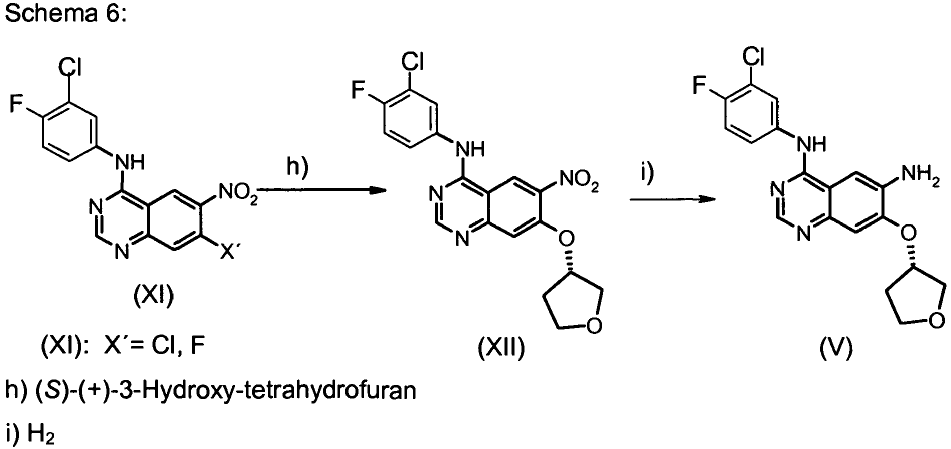

Die folgenden Beispiele sollen die Erfindung näher erläutern:

### Beispiele:

### Beispiel 1

### {[4-(3-Chlor-4-fluor-phenylamino)-7-((S)-tetrahydrofuran-3-yloxy)-chinazolin-6-ylcarbamoyl]-methyl}-phosphonsäure-diethylester

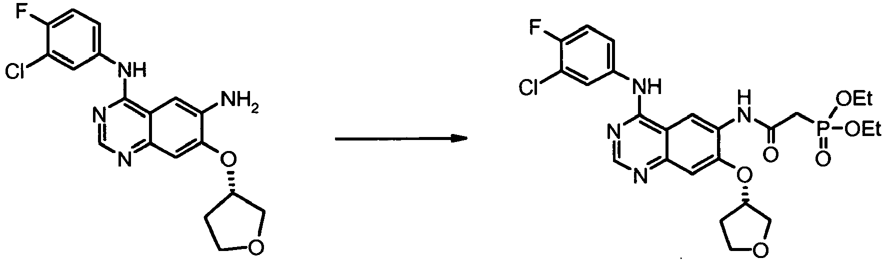

3,58 kg 1,1-Carbonyldiimidazol (22,16 mol) werden in 12,8 Liter Tetrahydrofuran vorgelegt und bei 40 °C mit 4,52 kg (22,16 mol) Diethylphosphonoessigsäure, gelöst in 6,5 Liter Tetrahydrofuran, versetzt. Es wird 30 Minuten lang bei 40 °C nachgerührt. Die so erhaltene Lösung wird als Lösung A bezeichnet.

6,39 kg (17,05 mol) N⁴-(3-Chlor-4-fluor-phenyl)-7-(tetrahydrofuran-3-yloxy)chinazolin-4,6-diamin in 26,5 Liter Tetrahydrofuran vorlegt und bei 40 °C mit der Lösung A versetzt und 2 Stunden bei 30 °C nachgerührt. Zur Suspension werden 64 Liter tert.-Butylmethylether gegeben und nach dem Abkühlen auf 20 °C wird der Niederschlag abzentrifugiert. Es wird mit einer Mischung aus 16 Liter Tetrahydrofuran und 16 Liter tert.-Butylmethylether und anschließend mit 32 Liter Wasser gewaschen und bei 50 °C getrocknet.
Ausbeute: 6,58 kg (69,8%) weiße Kristalle Gehalt: HPLC 99,1 FI%

### Beispiel 2

### (E)-4-Dimethylamino-but-2-ensäure-[4-(3-chlor-4-fluor-phenylamino)-7-((S)-tetrahydrofuran-3-yloxy)-chinazolin-6yl]-amid

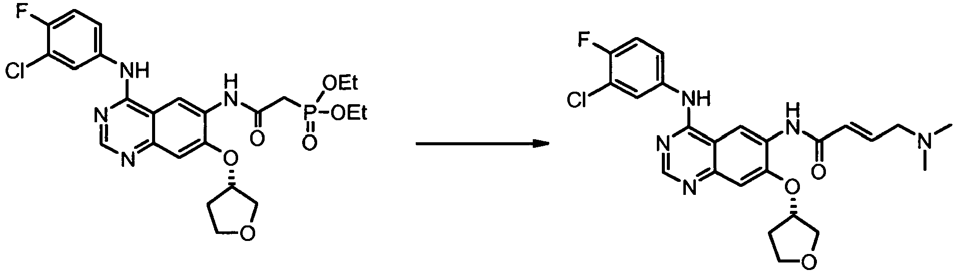

Zu 4,4 Liter Wasser werden 5,6 Liter 30 %-iger Salzsäure (53,17 mol) gegeben. Anschließend werden 4,28 kg 95 %-iges (Dimethylamino)-acetaldehyd-diethylacetal (26,59 mol) bei 30°C innerhalb 20 Minuten zugetropft. Die Reaktionslösung wird 8 Stunden bei 35°C nachgerührt, auf 5 °C abgekühlt und unter Argon aufbewahrt. Diese Lösung wird als Lösung B bezeichnet.

4,55 kg (68,06 mol) Kaliumhydroxid werden in 23,5 Liter Wasser gelöst und auf -5°C abgekühlt. Diese Lösung wird als Lösung C bezeichnet.

5,88 kg (10,63 mol) ((4-(3-Chlor-4-fluor-phenylamino)-7-(tetrahydrofuran-3-yloxy)-chinazolin-6-ylcarbamoyl)-methyl)-phosphonsäure-diethylester und 0,45 kg Lithiumchlorid (10,63 mol) werden in 23,5 Liter Tetrahydrofuran vorgelegt und auf -7 °C abgekühlt. Die kalte Lösung C wird innerhalb 10 Minuten zugegeben. Anschließend wird die Lösung B bei -7 °C innerhalb 1 Stunde zugegeben. Nach einstündigem Nachrühren bei -5 °C wird das Reaktionsgemisch auf 20°C erwärmt und mit 15 Liter Wasser versetzt. Nach dem Abkühlen auf 3 °C wird die Suspension abgesaugt, der Niederschlag mit Wasser gewaschen und getrocknet. Ausbeute: 5,21 kg roh 100 % Wassergehalt: 6,7 %

Die Kristallisation des Rohproduktes erfolgt mit Butylacetat / Methylcyclohexan Ausbeute: 78 % Reinheit HPLC 99,4 FI%, Wassergehalt 5,4 %

## Patentansprüche

1. Verfahren zur Herstellung einer Verbindung der Formel (I) 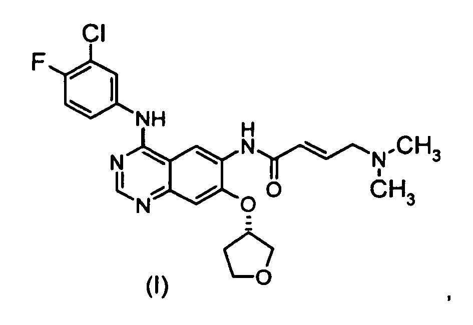 umfassend folgende Syntheseschritte:
a) Umsetzung einer Verbindung der Formel (V) 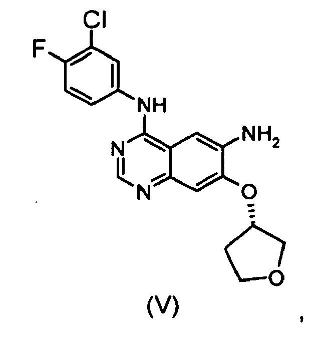 in geeigneten Lösungsmitteln nach entsprechender Aktivierung mit Di-(C₁₋₄-alkyl)-phosphonoessigsäure und
b) Umsetzung der so erhaltenen Verbindung der Formel (VI) 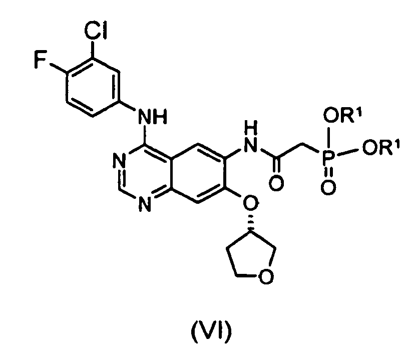 in
R¹ eine lineare oder verzweigte C₁₋₄-Alkylgruppe bedeutet,
mit dem Aldehyd der Formel 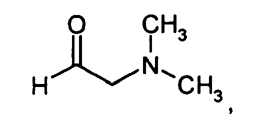 oder einem entsprechenden Aldehyd-Äquivalent unter Einsatz geeigneter organischer oder anorganischer Basen.

2. Verfahren zur Herstellung von 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin gemäß Anspruch 1, umfassend folgende Syntheseschritte:
a) Umsetzung von N⁴-(3-Chlor-4-fluor-phenyl)-7-(tetrahydrofuran-3-yloxy)-chinazoln-4,6-diamin in geeigneten Lösungsmitteln nach entsprechender Aktivierung mit Di-(C₁₋₄-alkyl)-phosphonoessigsäure und
b) Umsetzung des so erhaltenen {[4-(3-Chlor-4-fluor-phenylamino)-7-((*S*)-tetrahydrofuran-3-yloxy)-chinazolin-6-ylcarbamoyl]-methyl}phosphonsäure-dialkylesters mit dem *in situ* aus dem entsprechenden (Dimethylamino)-acetaldehyd-dialkylacetal hergestellten Aldehyd unter Einsatz geeigneter organischer oder anorganischer Basen.

3. Verfahren gemäß Anspruch 2, wobei als in Schritt a) Diethylphosphonoessigsäure als Reagenz verwendet wird.

4. Verfahren gemäß Anspruch 1 oder 2, **dadurch gekennzeichnet, dass** in Schritt b) DBU (1,5-Diazabicyclo[4.3.0]non-5-en), Natriumhydroxid oder Kaliumhydroxid als Base verwendet wird.

5. Verfahren gemäß Anspruch 4, **dadurch gekennzeichnet, dass** in Schritt b) Kaliumhydroxid als Base verwendet wird.

## Claims

1. Process for preparing a compound of general formula (VII) 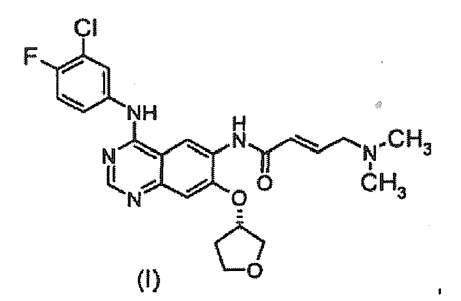 comprising the following synthesis steps:
a) reacting a compound of general formula (V) 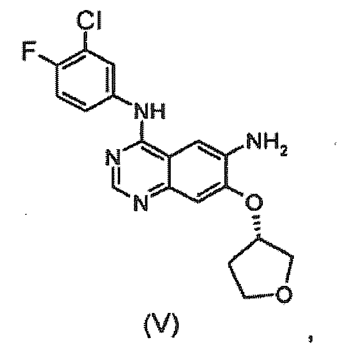 in suitable solvents after corresponding activation with di-(C₁₋₄-alkyl)-phosphonoacetic acid and
b) reacting the resulting compound of general formula (VI) 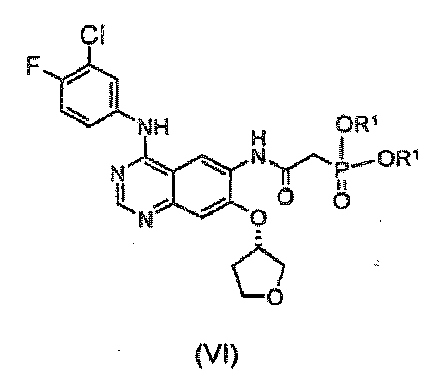 in
R¹ denotes a straight-chain or branched C₁₋₄-alkyl group,
with the aldehyde of formula 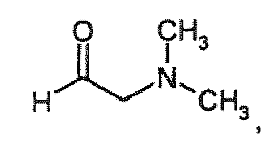 or a corresponding aldehyde equivalent, using suitable organic or inorganic bases.

2. Process for preparing 4-[(3-chloro-4-fluorophenyl)amino]-6-{[4-(N,N-dimethyl-amino)-1-oxo-2-buten-1-yl]amino}-7-((S)-tetrahydrofuran-3-yloxy)-quinazoline according to claim 1, comprising the following synthesis steps:
a) reacting N⁴-(3-chloro-4-fluoro-phenyl)-7-(tetrahydrofuran-3-yloxy)quinazoline-4,6-diamine in suitable solvents after corresponding activation with di-(C₁₋₄-alkyl)-phosphonoacetic acid and
b) reacting the resulting dialkyl {[4-(3-chloro-4-fluoro-phenylamino)-7-((*S*)-tetrahydrofuran-3-yloxy)-quinazolin-6-ylcarbamoyl]-methyl}-phosphonate with the aldehyde prepared *in situ* from the corresponding (dimethylamino)-acetaldehyde-dialkylacetal using suitable organic or inorganic bases.

3. Process according to claim 2, wherein as in step a) diethylphosphonoacetic acid is used as reagent.

4. Process according to claim 1 or 2, **characterised in that** in step b) DBU (1,5-diazabicyclo[4.3.0]non-5-ene), sodium hydroxide or potassium hydroxide is used as base.

5. Process according to claim 4, **characterised in that** in step b) potassium hydroxide is used as base.

## Revendications

1. Procédé de production d'un composé de formule (I) 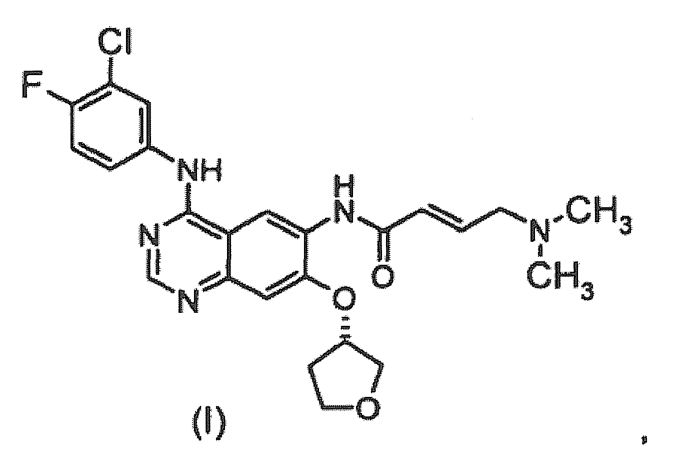 comprenant les étapes de synthèse suivantes :
a) réaction d'un composé de formule (V) 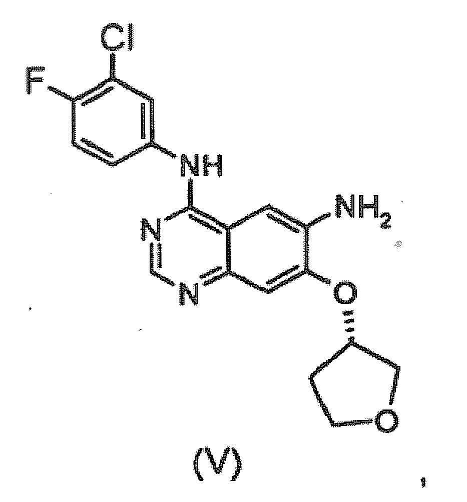 dans des solvants appropriés après activation correspondante avec de l'acide di-(alkyle en C₁ à C₄)-phosphono-acétique et
b) réaction du composé de formule (VI) ainsi obtenu 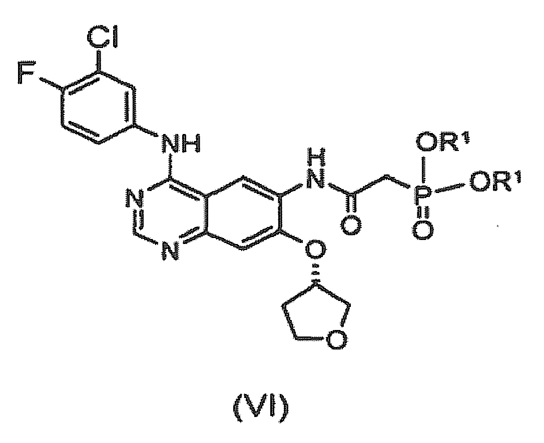 dans laquelle
R¹ désigne un groupe alkyle en C₁ à C₄ linéaire ou ramifié,
avec l'aldéhyde de formule 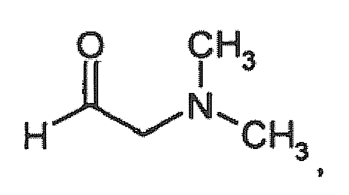 ou un équivalent d'aldéhyde correspondant en utilisant une base organique ou inorganique appropriée.

2. Procédé de production de 4-[(3-chloro-4-fluorophényl)amino]-6-{[4-(N,N-di-méthylamino)-1-oxo-2-butén-1-yl]amino)-7-((S)-tétrahydrofuran-3-yloxy)-quinazoline selon la revendication 1, comprenant les étapes de synthèse suivantes :
a) réaction de la N⁴-(3-chloro-4-fluoro-phényl)-7-(tétrahydrofuran-3-yloxy)-quinazolin-4,6-diamine dans des solvants appropriés après activation correspondante avec de l'acide di-(alkyle en C₁ à C₄)-phosphono-acétique et
b) réaction du dialkylester d'acide {[4-(3-chloro-4-fluoro-phénylamino)-7-((S)-tétrahydrofuran-3-yloxy)-quinazolin-6-ylcarbamoyl]-méthyl)-phosphonique ainsi obtenu avec l'aldéhyde produit *in situ* à partir de (diméthylamino)-acétaldéhyde-dialkylacétal correspondant en utilisant des bases organiques ou inorganiques appropriées.

3. Procédé selon la revendication 2, dans lequel on utilise comme à l'étape a), de l'acide diéthylphosphono-acétique comme réactif.

4. Procédé selon la revendication 1 ou 2, **caractérisé en ce que**, à l'étape b), on utilise du DBU (1,5-diazabicyclo[4.3.0]non-5-ène), de l'hydroxyde de sodium ou de l'hydroxyde de potassium comme base.

5. Procédé selon la revendication 4, **caractérisé en ce que**, à l'étape b) on utilise de l'hydroxyde de potassium comme base.
